(19) Europäisches Patentamt
European Patent Office
Office européen des brevets

(11) **EP 2 134 248 B1**

(12) **FASCICULE DE BREVET EUROPEEN**

(45) Date de publication et mention de la délivrance du brevet:
**14.10.2015 Bulletin 2015/42**

(21) Numéro de dépôt: **08762132.2**

(22) Date de dépôt: **20.02.2008**

(51) Int Cl.:
***A61B 3/103*** *(2006.01)*

(86) Numéro de dépôt international:
**PCT/FR2008/050285**

(87) Numéro de publication internationale:
**WO 2008/113937 (25.09.2008 Gazette 2008/39)**

(54) **DISPOSITIF DE MODULATION DE PHASE POUR UN INSTRUMENT OPHTALMIQUE, INSTRUMENTS OPHTALMIQUES EQUIPES DE CE DISPOSITIF, ET PROCEDE DE CALIBRATION ASSOCIE**

PHASENMODULATIONSVORRICHTUNG FÜR EIN OPHTHALMISCHES INSTRUMENT, MIT EINER SOLCHEN VORRICHTUNG AUSGESTATTETE OPHTHALMISCHE INSTRUMENTE UND KALIBRIERUNGSVERFAHREN DAFÜR

PHASE MODULATION DEVICE FOR AN OPHTHALMIC INSTRUMENT, OPHTHALMIC INSTRUMENTS EQUIPPED WITH SUCH DEVICE, AND RELATED CALIBRATION METHOD

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **21.02.2007 FR 0701249**

(43) Date de publication de la demande:
**23.12.2009 Bulletin 2009/52**

(73) Titulaire: **Imagine Eyes**
**91400 Orsay (FR)**

(72) Inventeur: **LEVECQ, Xavier**
**F-91190 Gif sur Yvette (FR)**

(74) Mandataire: **Pontet Allano & Associes**
**Parc Les Algorithmes, Bâtiment Platon**
**CS 70003 Saint-Aubin**
**91192 Gif-sur-Yvette Cedex (FR)**

(56) Documents cités:
**WO-A-2005/089635      FR-A1- 2 866 551**
**US-B2- 6 964 480**

• **IROSHNIKOV NIKITA G ET AL: "Adaptive optics in ophthalmology" PROC SPIE INT SOC OPT ENG; PROCEEDINGS OF SPIE - THE INTERNATIONAL SOCIETY FOR OPTICAL ENGINEERING; INTERNATIONAL CONFERENCE ON LASERS, APPLICATIONS, AND TECHNOLOGIES 2005: LASER TECHNOLOGIES FOR ENVIRONMENTAL MONITORING AND ECOLOGICAL APPLICATIONS,, vol. 6284, 2006, XP002455689**

## Description

### Domaine technique

La présente invention concerne un dispositif de modulation de phase pour un instrument ophtalmique utilisant un faisceau lumineux interagissant avec un oeil. Elle vise également des instruments ophtalmiques implémentant un tel dispositif, ainsi qu'un procédé de calibration pour ces instruments ophtalmiques.

Le domaine de l'invention est plus particulièrement celui des simulateurs de correction visuelle et des appareils d'imagerie ophtalmique, notamment d'imagerie rétinienne, à haute résolution.

### Etat de la technique antérieure

Les instruments ophtalmiques, tels que par exemple les instruments d'imagerie rétinienne ou de traitement optique par laser sur la rétine, fonctionnent avec un faisceau principal destiné à traverser les divers éléments optiques (cornée, cristallin,...) dont est formé l'oeil d'un patient, soit en faisceau incident dans l'oeil (cas du traitement optique de la rétine), soit en faisceau émergent de l'oeil (cas de l'imagerie rétinienne).

Dans tous les cas, les aberrations des différents éléments optiques de l'oeil entachent d'aberrations le front d'onde du faisceau principal, ce qui dégrade la qualité de l'instrument optique. Ainsi dans le cas de l'imagerie rétinienne, l'image perd en résolution et, dans le cas du traitement optique de l'oeil, la qualité de focalisation du laser sur la rétine est dégradée.

Il est connu d'associer à ces instruments ophtalmiques un système de correction des aberrations de l'oeil permettant de corriger le front d'onde du faisceau principal, c'est-à-dire de donner à la phase du faisceau optique la forme la plus proche possible d'une forme prédéterminée permettant d'obtenir les performances optimales de l'instrument.

Un tel système peut aussi être utilisé dans un instrument ophtalmique de type instrument de simulation de vision, dont le but est de représenter à un patient les effets de différentes corrections (verres correcteurs, lentilles de contact, chirurgie réfractive) en lui faisant "voir" une image, le faisceau principal incident dans l'oeil du patient étant alors corrigé des aberrations oculaires et/ou présentant les effets optiques induits par le phénomène que l'on souhaite simuler.

Un tel système de correction des aberrations, décrit par exemple dans la demande de brevet FR 2 866 551, comprend classiquement des moyens de mesure des aberrations de l'oeil, de type analyseur Shack Hartmann, et un dispositif optique de modulation de la phase du front d'onde du faisceau principal, de type miroir déformable ou modulateur spatial de lumière et commandé pour corriger le front d'onde en fonction des aberrations de l'oeil mesurées. Dans ces systèmes, la commande du dispositif optique de modulation est calculée en fonction d'une modulation de la phase que l'on désire effectuer sur le faisceau principal.

Lorsqu'il s'agit d'un équipement de simulation visuelle, fonctionnant en boucle fermée, on est généralement confronté au problème de devoir envoyer de la lumière dans l'oeil d'un patient, ce qui a pour effet de l'éblouir et ainsi de perturber le processus de simulation, et aussi de créer une sensation de gène oculaire.

Pour pallier ce problème d'éblouissement en boucle fermée, on pourrait certes employer un faisceau incident dans un domaine spectral non visible par l'oeil, typiquement dans l'infrarouge, mais on se trouverait alors dans le cas d'une dépendance aux effets de chromatisme liée à un écart de longueur d'onde important entre la mesure des aberrations (réalisée dans les spectre infra rouge) et la stimulation visuelle (réalisée dans le spectre visible). Ou pourrait aussi envisager la commande des moyens de modulation en boucle ouverte (sans rétroaction). Dans ce cas, aucune lumière n'est envoyée dans l'oeil du patient mais, en corollaire, aucune mesure de front d'onde n'est possible car aucune lumière ne revient de l'oeil. Une telle approche se heurte à la non perfection (problème de linéarité, hystérésis, dérive en température) des moyens de modulation. Les erreurs générées sur la modulation par ces défauts ne sont pas compensées par une boucle de rétroaction (boucle fermée) et dégradent ainsi les résultats de la correction ou de la simulation.

Par ailleurs, un autre problème réside dans la nécessité de prévoir une phase d'apprentissage ou de calibration du procédé d'optique adaptative, dont la périodicité dépend typiquement de l'exigence de précision et de stabilité pour une application donnée. En pratique, cette phase d'apprentissage est réalisée en utilisant un oeil artificiel à l'utilisation contraignante : il faut que cet oeil artificiel soit de très bonne qualité optique et nécessite un alignement soigneux devant l'instrument.

Se pose aussi le problème de la maîtrise des aberrations du système optique lui-même. En effet, dans de nombreuses applications dans lesquelles on veut effectuer des mesures des aberrations de l'oeil de façon à faire de la correction ou de la simulation, il est important de maîtriser les aberrations du système optique mis en oeuvre car il faut être capable de discriminer, d'une part, les aberrations du système optique du système ophtalmique et, d'autre part, les aberrations induites par l'oeil. Une simple mesure des aberrations du système réalisée lors de son montage n'est, en général, pas suffisante pour assurer la connaissance de ces aberrations sur une longue période (typiquement plusieurs mois) à cause de leur dépendance à la température. En effet, l'élément déphaseur est, en général, un élément optique sensible à la température de son environnement. Ainsi, la connaissance ou la maîtrise des aberrations du système optique de l'appareil nécessite une mesure dans ses conditions d'utilisation. En pratique, cette mesure doit être faite très régulièrement et nécessite, dans l'état de l'art, l'emploi d'un oeil artificiel.

On peut aussi être confronté à la nécessité d'ajouter des corrections supplémentaires lorsqu'on souhaite simuler des objets de phase, telles qu'une lentille ou un implant intraoculaire. Se pose là encore la nécessité de la maîtrise des aberrations du dispositif ophtalmique de simulation. En effet, il faut être capable de maîtriser les aberrations du système optique du dispositif ophtalmique pour être capable de générer, dans la pupille de l'oeil, une modulation de phase qui représente ce qu'on l'on souhaite modulé dénué des aberrations du système optique du dispositif ophtalmique.

On connait aussi le document WO 2005/089 635, qui décrit un dispositif ophtalmique comprenant des moyens pour positionner un miroir adaptatif dans sa « position zéro ».

Le but de l'invention est de remédier à ces problèmes en proposant un dispositif ophtalmique dans lequel un processus complet d'optique adaptative en boucle fermée, comprenant aussi une phase d'apprentissage, puisse être aisément implémenté, sans avoir recours à l'utilisation fréquente d'un oeil artificiel, sans gène pour les patients et dans conditions optimales de maîtrise des aberrations, malgré des dérives de la température ambiante, des non-linéarités et des phénomènes d'hystérésis sur les modulateurs de phase.

**Exposé de l'invention**

Cet objectif est atteint avec un dispositif de modulation de phase mis en oeuvre dans un instrument ophtalmique utilisant un faisceau lumineux principal interagissant avec un oeil, ce dispositif comprenant :

- des moyens pour moduler la phase du front d'onde dudit faisceau lumineux principal,
- des moyens pour commander lesdits moyens de modulation de phase selon une consigne de modulation, et
- des moyens pour analyser la modulation ainsi effectuée de la phase du front d'onde dudit faisceau lumineux principal.

Suivant l'invention, ce dispositif comprend en outre des moyens pour émettre un faisceau lumineux secondaire, des moyens pour diriger le faisceau lumineux secondaire le long d'un chemin optique vers les moyens de modulation puis vers les moyens d'analyse, ce chemin optique ne passant par l'oeil, et les moyens de commande et les moyens d'analyse recevant le faisceau lumineux secondaire modulé coopèrent, au cours d'une phase dite d'apprentissage, pour fournir des données de réponse des moyens de modulation de phase à un ensemble de consignes de modulation prédéterminées.

On dispose ainsi d'un dispositif de modulation de phase permettant de réaliser des calibrations périodiques des instruments ophtalmique, sans qu'il soit nécessaire d'éclairer l'oeil d'un patient ou d'utiliser un oeil artificiel.

Il est à noter que le faisceau lumineux secondaire mis en oeuvre dans le dispositif de modulation de phase selon l'invention peut être émis à partir d'une source lumineuse qui est soit interne, soit externe à l'instrument ophtalmique ainsi équipé. Ce faisceau lumineux secondaire peut être complètement interne à l'instrument ophtalmique, mais on peut tout aussi bien prévoir qu'une partie du trajet optique de ce faisceau secondaire soit effectuée à l'extérieur de l'instrument.

Dans le dispositif de modulation de phase selon l'invention, le faisceau lumineux secondaire est dirigé selon un chemin optique ne passant par l'oeil examiné. Toutefois, on peut aussi prévoir, sans pour autant sortir du cadre de la présente invention, qu'une fraction de ce faisceau lumineux secondaire, qui soit par exemple issue d'une lame séparatrice, suive un autre chemin optique qui, lui, passe par l'oeil.

Dans le cas d'un instrument ophtalmique dans lequel les moyens de modulation de phase comprennent un miroir déformable sous l'action d'une pluralité d'actionneurs, les moyens de commande et les moyens d'analyse de front d'onde coopèrent pendant la phase d'apprentissage pour mettre en mouvement successivement chaque actionneur et pour enregistrer, pour chaque actionneur, la réponse des moyens d'analyse de front d'onde.

Le dispositif de modulation de phase selon l'invention peut en outre avantageusement comprendre des moyens pour modifier la commande appliquée aux moyens de modulation, de manière à réduire un écart entre la consigne de modulation et la modulation effectuée sur le faisceau secondaire.

Les moyens de modulation sont de préférence agencés pour moduler simultanément, selon une consigne de modulation, la phase du front d'onde du faisceau principal et la phase du front d'onde du faisceau secondaire.

Dans une première gamme d'utilisation du dispositif de modulation de phase selon l'invention, correspondant à des instruments de simulation visuelle, celui-ci comprend en outre des moyens pour projeter sur la rétine de l'oeil une image d'une cible. Les rayons lumineux issus de cette cible peuvent avantageusement constituer le faisceau principal.

Les moyens de modulation sont alors commandés pour simuler sur le faisceau principal une correction statique solidaire de l'oeil, comme une chirurgie réfractive, ou une correction par une lentille de contact, un verre de lunette ou un implant oculaire.

Une seconde gamme d'utilisation du dispositif de modulation de phase selon l'invention correspond à des instruments d'imagerie rétinienne, à haute résolution comprenant en outre des moyens pour éclairer la rétine de l'oeil, des moyens pour détecter un faisceau lumineux émergent de l'oeil et pour former une image de la rétine, ce faisceau émergent constituant le faisceau principal.

Une troisième gamme d'utilisation d'un dispositif de modulation de phase selon l'invention, correspond à des instruments de traitement optique de la rétine de l'oeil comprenant une source laser pour l'émission du faisceau principal, et des moyens pour focaliser le faisceau principal sur la rétine de l'oeil.

On peut aussi prévoir une utilisation d'un dispositif de modulation de phase selon l'invention dans un instrument de mesure des aberrations de l'oeil, comprenant des moyens pour mesurer des aberrations de l'oeil susceptibles de perturber le faisceau principal, et des moyens pour calculer la consigne de modulation en fonction de valeurs mesurées des aberrations de l'oeil.

Dans un tel instrument, les moyens de mesure des aberrations de l'oeil comprennent des moyens d'émission d'un faisceau d'éclairage pour former sur la rétine de l'oeil une source lumineuse secondaire, cette source secondaire émettant un faisceau qui émerge de l'oeil, et des moyens pour guider ce faisceau jusqu'à des moyens de mesure de la phase du front d'onde de ce faisceau.

Les instruments ophtalmiques selon l'invention peuvent en outre comprendre des moyens pour mesurer des mouvements de l'oeil, et des moyens pour calculer la consigne de modulation en fonction de valeurs mesurées des mouvements de l'oeil.

Ces moyens pour mesurer des mouvements de l'oeil comprennent par exemple des moyens pour mesurer un déplacement latéral de la pupille de l'oeil par rapport à une position prédéterminée, des moyens d'éclairage de la pupille de l'oeil, un détecteur matriciel, et un objectif formant l'image de la pupille de l'oeil sur le détecteur.

Suivant un autre aspect de l'invention, il est proposé un procédé pour calibrer un appareil ophtalmique utilisant un faisceau lumineux principal interagissant avec un oeil et dans lequel sont mises en oeuvre : (i) une modulation de la phase du front d'onde dudit faisceau lumineux principal par des moyens de modulation de phase et (ii) une analyse de ladite phase ainsi modulée du front d'onde par des moyens d'analyse de la phase.

Ce procédé est caractérisé en ce qu'il comprend, pendant une phase dite d'apprentissage :

- une émission d'un faisceau lumineux secondaire qui est soumis à ladite modulation de phase puis à ladite analyse de phase, et
- une séquence de commandes prédéterminées desdits moyens de modulation de phase, et d'enregistrements de données d'analyse de phase en réponse auxdites commandes de modulation,

et en ce que le faisceau lumineux secondaire est dirigé le long d'un chemin optique vers lesdits moyens de modulation et vers lesdits moyens d'analyse de la phase, ledit chemin optique ne passant pas par l'oeil.

Le procédé selon l'invention peut être mis en oeuvre dans un appareil dans lequel les moyens de modulation de phase comprennent un miroir déformable sous l'action d'une pluralité d'actionneurs. La séquence de commandes prédéterminées comprend des commandes pour mettre en mouvement successivement chaque actionneur et pour enregistrer, pour chaque actionneur, la réponse desdits moyens d'analyse de front d'onde.

Il aurait pu paraître évident, pour limiter la gêne visuelle d'un patient tout en s'assurant de la bonne commande d'un dispositif de modulation de phase, d'améliorer le procédé d'asservissement décrit dans le document FR2 866 551 en utilisant pour une mesure des aberrations de l'oeil une source d'éclairage non visible par l'oeil, typiquement à une longueur d'onde infrarouge supérieure à 900 nanomètres. Cependant, du fait du chromatisme de l'oeil, l'asservissement des moyens de modulation réalisé dans l'infra rouge n'aurait pas été forcément valable pour le faisceau principal si celui-ci avait une longueur d'onde différente, typiquement dans le spectre visible. De plus, l'utilisation d'un faisceau secondaire interagissant avec l'oeil limite nécessairement le flux disponible à la mesure en raison des normes de sécurité oculaires. Cela implique que cette mesure soit réalisée avec un taux de récurrence faible ou avec un analyseur de phase présentant un nombre réduit de points de mesure. Dans ces deux cas, la qualité de l'asservissement est dégradée.

L'invention permet de manière astucieuse d'asservir la commande des moyens de modulation, car dans le cas préférentiel où une longueur d'onde du faisceau secondaire est sensiblement égale à une longueur d'onde du faisceau principal, il permet en même temps de s'affranchir de problèmes d'achromatisme de l'oeil et de l'ensemble des éléments optiques utilisés dont les moyens de modulation de la phase, en particulier dans le cas où les moyens de modulation comprennent un modulateur de phase à cristaux liquide.

De manière préférentielle, une longueur d'onde du faisceau secondaire est sensiblement égale à une longueur d'onde du faisceau principal.

Le procédé de calibration selon l'invention, mis en oeuvre dans un instrument ophtalmique comprenant une première source de génération d'un faisceau lumineux principal pour éclairer la rétine d'un oeil et une seconde source de génération d'un faisceau lumineux secondaire, peut avantageusement comprendre :

- une étape de mesure des aberrations du système optique de l'instrument ophtalmique, ladite première source d'éclairage de la rétine étant allumée,
- une étape de détermination de la position de la pupille de l'oeil pendant l'étape de mesure des aberrations,
- une étape d'extinction de la première source d'éclairage de la rétine,
- une étape de mise en route de la seconde source de génération du faisceau secondaire, ce faisceau secondaire étant utilisé dans la phase d'apprentissage, et
- une étape d'application de commandes de correction et/ou de simulation sur les moyens de modulation de phase.

L'ensemble de ces étapes peut être automatisé de sorte que l'opérateur d'un instrument ophtalmique implémentant un tel procédé selon l'invention puisse disposer d'une calibration automatique, éventuellement périodique, de cet instrument.

On peut aussi prévoir que le procédé de calibration selon l'invention comprenne en outre une mise en oeuvre d'une cible de fixation, telle qu'un micro-afficheur, sur laquelle des images ou tests visuels peuvent être affichés, cette cible de fixation étant prévue pour procurer à un patient une mesure subjective de l'effet de la correction et/ou de la simulation.

Ce procédé peut aussi inclure, préalablement à l'étape de mesure des aberrations, une étape initiale d'apprentissage et une étape de maîtrise des aberrations du système optique de l'instrument ophtalmique.

L'étape de maîtrise des aberrations du système optique comprend par exemple un asservissement des moyens de modulation de phase, qui peuvent être réalisés sous la forme d'un miroir déformable par une pluralité d'actionneur, par rapport à une consigne déterminée de façon à corriger les aberrations du système optique.

**Description des figures et modes de réalisation**

D'autres avantages et particularités de l'invention apparaîtront à la lecture de la description détaillée de mises en oeuvre et de modes de réalisation nullement limitatifs, et des dessins annexés suivants :

- la figure 1 illustre un dispositif de modulation de phase selon l'invention, intégré dans un instrument de simulation de vision et mettant en oeuvre des étapes du procédé selon l'invention,
- la figure 2 illustre le simulateur de vision de la figure 1 mettant en oeuvre d'autres étapes du procédé selon l'invention,
- la figure 3 illustre un dispositif de modulation de phase selon l'invention, intégré dans un instrument d'imagerie rétinienne et mettant en oeuvre des étapes du procédé selon l'invention,
- la figure 4 illustre le dispositif d'imagerie de la figure 3 mettant en oeuvre d'autres étapes du procédé selon l'invention, et
- la figure 5 illustre les faisceaux lumineux mis en oeuvre lors d'une phase d'apprentissage dans le simulateur de vision des figures 1 et 2.

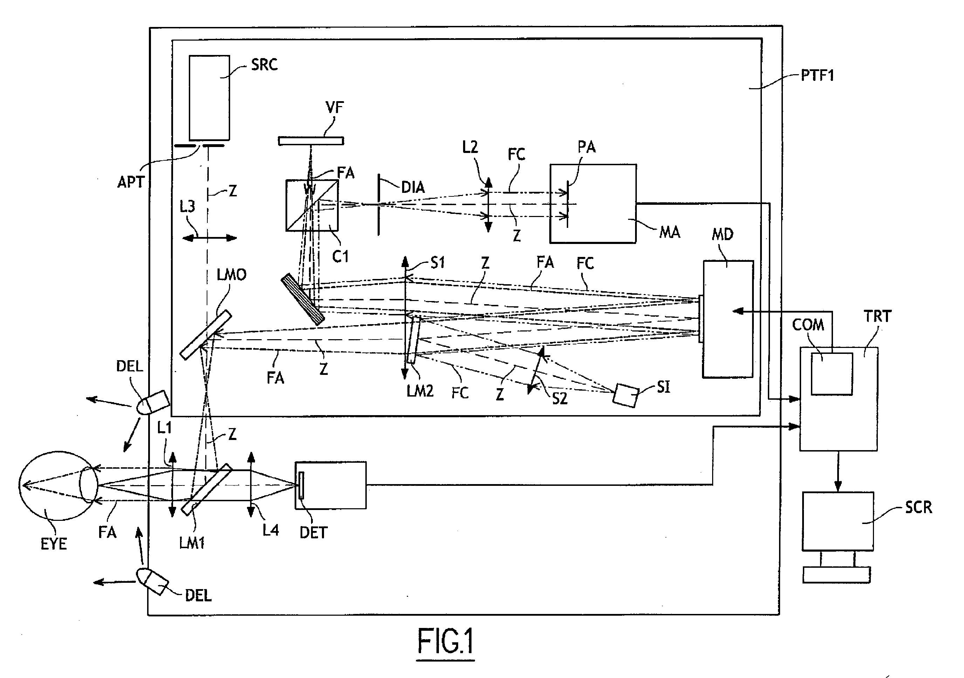

Pour simplifier les figures 1 à 5, chaque faisceau lumineux qui y est représenté est schématisé par deux rayons lumineux bordant ledit faisceau.

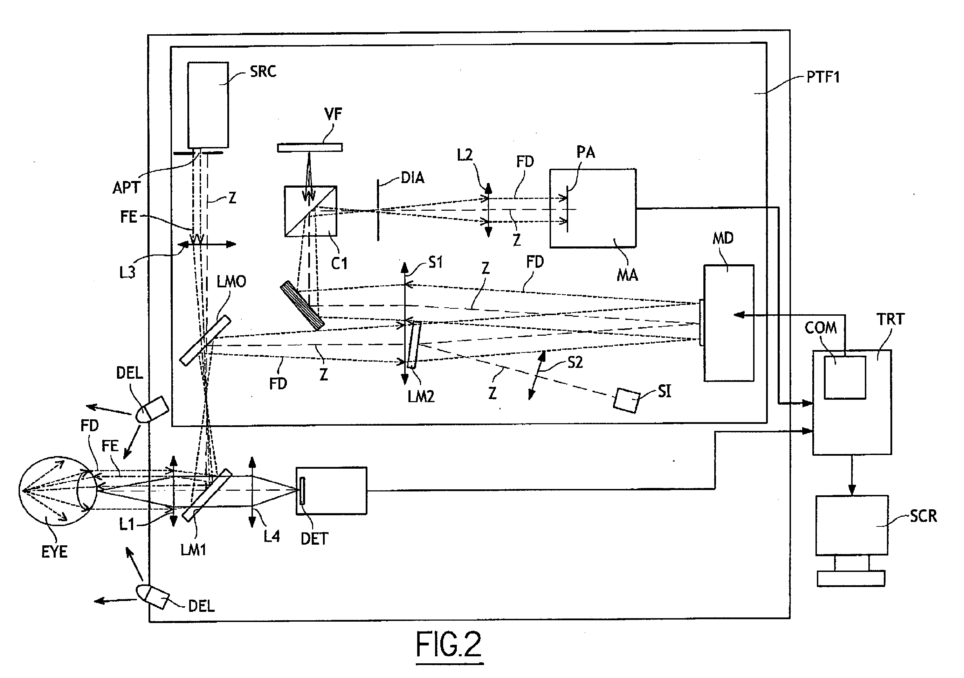

On va tout d'abord décrire, en référence aux figures 1 et 2, un dispositif de modulation de phase selon l'invention intégré dans un instrument de simulation de vision et mettant en oeuvre un procédé selon l'invention.

Un tel dispositif comprend une voie de fixation incluant une source lumineuse VF qui, lorsqu'elle est allumée, émet une image test destinée à être vue par l'oeil EYE d'un patient. Cette source lumineuse comprend typiquement un micro afficheur (de technologie OLED ou LCD ou MEMS) sur lequel peut être affiché différentes images ou tests visuels. L'image test est constituée d'un ensemble de rayons lumineux qui forment le faisceau lumineux principal FA du dispositif. Le faisceau lumineux principal FA traverse une lame séparatrice C1, puis est guidé vers des moyens MD pour moduler la phase du front d'onde du faisceau principal. La lame séparatrice C1, comme toutes les autres lames séparatrices décrites par la suite, peut typiquement consister en une lame semi-réfléchissante ou en un filtre dichroïque. Les moyens de modulation MD interceptent le faisceau principal FA. Les moyens de modulation sont précédés d'un système optique S1 agencé pour que le faisceau principal FA soit un faisceau de lumière parallèle lorsqu'il est intercepté par les moyens de modulation MD. Les moyens de modulation sont reliés à une unité de commande COM elle-même reliée à des moyens de traitement TRT. Ces moyens de commande COM et de traitement TRT comprennent typiquement une unité centrale d'un ordinateur ou une simple carte électronique. Les moyens de commande COM appliquent aux moyens de modulation MD une commande qui permet de modifier localement la phase du front d'onde du faisceau principal FA. La commande est calculée par les moyens de traitement à partir d'une consigne de modulation qui correspond à la modulation que l'on désire voir effectuée par les moyens de modulation sur le faisceau principal ou tout autre faisceau intercepté par ces moyens.

Les moyens optiques de modulation MD comprennent un miroir déformable constitué d'une surface réfléchissante pouvant être déformée par un ensemble d'actionneurs sur laquelle le faisceau principal est incident, positionné sensiblement dans un plan conjugué de la pupille de l'oeil, chaque actionneur étant commandé par l'unité de commande pour moduler la phase locale du front d'onde du faisceau principal. Il est également possible d'utiliser un modulateur spatial de lumière (ou SLM selon l'abréviation de l'expression anglo-saxonne « Spatial Light Modulator »), par exemple réalisé par une matrice de valves à cristaux liquides.

Après que sa phase ait été corrigée par le miroir déformable, le faisceau principal FA poursuit son trajet optique. Il est réfléchi par des lames séparatrices LM0 puis LM1 avant d'être focalisé par une lentille L1 sur la rétine de l'oeil. Le chemin optique du faisceau principal FA, qui part de la source VF jusqu'à la rétine de l'oeil EYE est illustré sur la figure 1 par deux lignes pointillées.

Le dispositif comprend en outre des moyens SI pour émettre un faisceau lumineux de mesure FC. Lorsqu'il est émis, le faisceau lumineux de mesure est guidé vers les moyens de modulation MD, typiquement par une lame séparatrice LM2 située sur le chemin optique du faisceau principal entre les moyens de modulation MD et la lame séparatrice LMO. La lame séparatrice permet de superposer sensiblement les portions des chemins optiques du faisceau secondaire et du faisceau principal situées entre la lame séparatrice LM2 et la lame séparatrice C1. Les moyens de modulation MD interceptent donc le faisceau secondaire FC. La lame séparatrice LM2 est précédée d'un système optique S2 agencé pour que le faisceau secondaire FC soit un faisceau de lumière parallèle lorsqu'il est intercepté par les moyens de modulation MD. Les moyens de modulation sont donc agencés pour corriger ou moduler localement la phase du front d'onde du faisceau secondaire FC selon la consigne de modulation correspondant à la commande appliquée aux moyens de modulation MD.

[0049]  - Après que sa phase ait été modulée par le miroir déformable, le faisceau secondaire FC poursuit son trajet optique. Il traverse le système optique S1, est séparé du chemin optique du faisceau principal FA en étant réfléchi par la lame séparatrice C1, puis traverse une lentille L2 et est dirigé vers des moyens de mesure optique MA agencés pour mesurer, dans un plan de mesure donné PA, la phase du front d'onde du faisceau secondaire. Les moyens de mesure MA, par exemple un analyseur de type Hartmann ou Shack-Hartmann, sont reliés aux moyens de traitement TRT qui, de manière connue, établissent la cartographie en phase du faisceau secondaire. Une représentation de cette cartographie peut être affichée sur un écran SCR.

Le faisceau secondaire FC est guidé le long d'un chemin optique de sorte qu'il n'interagisse pas avec l'oeil EYE. Le chemin optique du faisceau secondaire FC, qui part des moyens d'émission SI et va jusqu'aux moyens de mesure MA, est illustré sur la figure 1 par lignes cadratin-point-point. La longueur d'onde du faisceau secondaire FC est sensiblement égale à la longueur d'onde du faisceau principal FA, ce qui permet de s'affranchir de problèmes de chromatisme de l'oeil et de chromatisme des éléments optiques de l'instrument, notamment du chromatisme éventuel des moyens de modulation de la phase MD. Pour que l'image test soit visible par l'oeil, la longueur d'onde du faisceau secondaire FC et celle du faisceau principal FA sont situées dans le visible, c'est-à-dire approximativement de 400 à 700 nanomètres, ou dans le proche infrarouge.

Les moyens de traitement TRT sont en outre équipés de moyens pour mesurer la modulation de phase effectuée sur le faisceau secondaire par les moyens de modulation MD, et notamment pour comparer la modulation de phase effectuée sur le faisceau secondaire et la consigne de modulation, ladite consigne correspondant à la modulation que l'on désire voir effectuée par les moyens de modulation sur tout faisceau intercepté par ces derniers. Les moyens de traitement TRT sont reliés à l'unité de commande et comprennent des moyens pour modifier la commande appliquée aux moyens de modulation, en particulier si la modulation effectuée diffère de la consigne de modulation. De préférence, la modification est réalisée pour minimiser un écart entre la consigne de modulation et la modulation effectuée sur le faisceau secondaire, de préférence jusqu'à ce que la modulation effectuée sur le faisceau secondaire soit égale ou du moins sensiblement égale à la consigne de modulation. Les moyens de modification de la commande constituent donc un système de rétroaction, ayant pour valeurs d'entrée la consigne de modulation et la mesure de la modulation de phase effectuée sur le faisceau secondaire par les moyens de modulation, et comme valeur de sortie la commande calculée à partir des valeurs d'entrée et appliquée aux moyens de modulation. La mesure de la modulation de phase effectuée sur le faisceau secondaire par les moyens de modulation peut être calculée par les moyens de traitement comme la différence entre la mesure, par les moyens de mesure MA, de la phase du front d'onde du faisceau secondaire, et une valeur, mémorisée dans les moyens de traitement, de la phase du front d'onde du faisceau secondaire tel qu'il est émis.

Les moyens de traitement TRT comprennent des moyens pour déterminer la commande en mettant en oeuvre un algorithme de calcul comprenant comme variable la consigne de modulation. Les moyens pour modifier la commande comprennent des moyens pour calibrer les moyens de détermination de la commande, et notamment pour calibrer des paramètres utilisés dans l'algorithme de calcul, tels des paramètres qui ne dépendent pas de manière simple ou linéaire de la consigne de modulation ou des paramètres qui dépendent de la température ambiante.

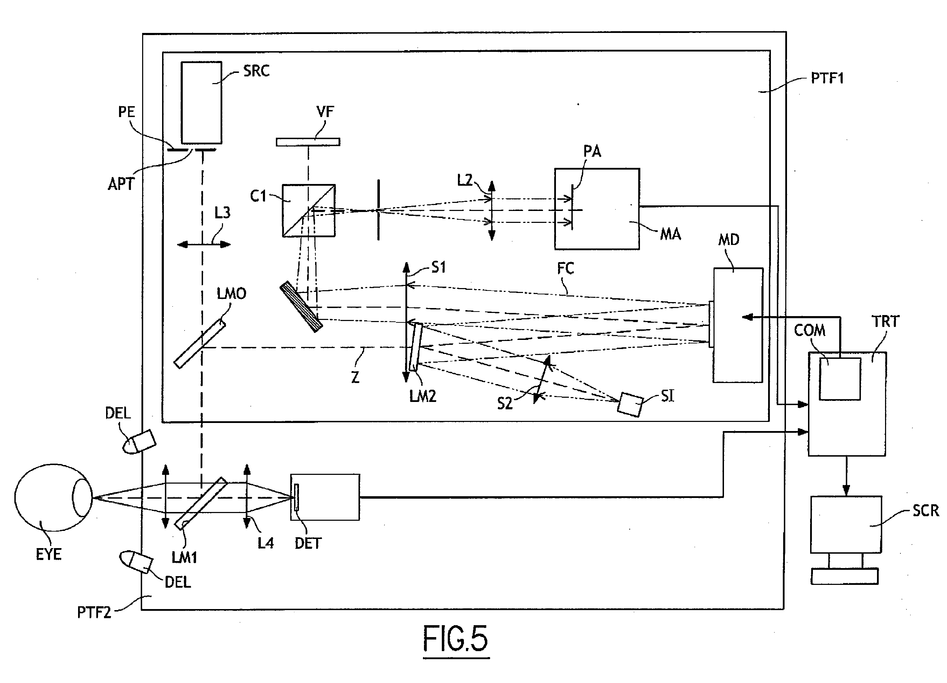

En référence à la figure 5, la phase d'apprentissage, ou de calibration de boucle d'optique adaptative, consiste à faire apprendre au logiciel comment est modulée la phase sous l'action de chaque actionneur ou combinaison d'actionneurs. Cette calibration peut être considérée comme une constante du système mais l'expérience montre qu'il préférable, pour garder des performances optimales, d'effectuer cette phase d'apprentissage régulièrement.

Une possibilité simple mais efficace de réalisation pratique de cette phase d'apprentissage consiste à faire bouger, actionneur par actionneur le miroir, et d'enregistrer, pour chaque actionneur, la réponse de l'analyseur.

Ainsi, à l'issue de cette phase d'apprentissage on obtient la relation :

$$MI \cdot V = P$$

où

MI est une matrice de n (nombre d'actionneurs) colonnes et m (nombres de microlentilles éclairées x2) lignes. La colonne i contient l'information en pentes locales de l'influence de l'actionneur i.

Les pentes sont rangés arbitrairement : d'abord les pentes locales x, puis les pentes locales y. Un élément du vecteur est une pentes locales sous une sous pupille.

V est le vecteur de tensions de commande du miroir déformable

P est le vecteurs des pentes x et y au foyer de chaque sous pupille.

A l'issue de cette phase on est capable de prédire ce que verra l'analyseur si on applique au miroir une commande V.

Ce que l'on souhaite pour la correction, c'est l'inverse : on veut connaître la commande à appliquée au miroir en fonction du vecteur des pentes x et y mesurées. En fait on veut une relation du type : V = MC.P

On remarque que MC est en fait l'inverse de MI. On obtient donc MC par inversion numérique de MI.

Un mode de réalisation de procédé selon l'invention mis en oeuvre dans le simulateur de vision selon l'invention illustré sur les figures 1 et 2 comprend les étapes suivantes :

- une commande des moyens de modulation selon la consigne de modulation,
- une émission du faisceau lumineux de mesure FC, la source SI étant allumée,
- une modulation, selon la consigne de modulation, de la phase du front d'onde du faisceau secondaire par les moyens de modulation MD,
- une mesure de la modulation de phase effectuée par les moyens de modulation sur le faisceau secondaire, et
- si la modulation de phase effectuée par les moyens de modulation sur le faisceau secondaire est sensiblement égale à la consigne de modulation, un maintien de la commande appliquée aux moyens de modulation, ou
- si la modulation de phase effectuée par les moyens de modulation sur le faisceau secondaire diffère de la consigne de modulation, une modification de la commande appliquée aux moyens de modulation, comprenant une calibration des moyens pour déterminer la commande.

Le premier mode de réalisation de procédé selon l'invention permet ainsi de calibrer le dispositif selon l'invention, sans utiliser d'oeil artificiel, et en l'absence du faisceau principal FA.

Un deuxième mode de réalisation de procédé selon l'invention, mis en oeuvre dans le simulateur selon l'invention et illustré sur la figure 1, comprend les étapes suivantes :

- une commande des moyens de modulation selon la consigne de modulation,
- un positionnement d'un oeil à examiner en sortie du simulateur selon l'invention,

- une émission du faisceau lumineux de mesure FC,
- une émission du faisceau lumineux principal FA,
- une modulation, selon la consigne de modulation, de la phase du front d'onde du faisceau secondaire par les moyens de modulation MD,
- une modulation, selon une consigne de modulation, de la phase du front d'onde du faisceau lumineux principal FA interagissant avec l'oeil EYE, les modulations des faisceaux principal et de mesure étant simultanées,
- une mesure de la modulation de phase effectuée par les moyens de modulation sur le faisceau secondaire, et
- si la modulation de phase effectuée par les moyens de modulation sur le faisceau secondaire est sensiblement égale à la consigne de modulation, un maintien de la commande appliquée aux moyens de modulation, ou
- si la modulation de phase effectuée par les moyens de modulation sur le faisceau secondaire diffère de la consigne de modulation, une modification de la commande appliquée aux moyens de modulation.

La modification de la commande peut comprendre une calibration des moyens pour déterminer la commande. La modification de la commande est effectuée de manière à réduire un écart entre la consigne de modulation et la modulation de phase effectuée sur le faisceau secondaire. Ainsi, un dispositif et un procédé selon l'invention permettent de vérifier puis de s'assurer que la modulation de phase effectuée sur le faisceau secondaire et donc sur le faisceau principal correspond à la consigne de modulation, quelle que soient les dérives de la température ambiante et la consigne de modulation.

Les moyens de traitement comprennent des moyens pour générer la consigne de modulation, de sorte que les moyens de modulation, en modulant le faisceau principal selon la consigne de modulation, simule sur le faisceau principal une correction statique solidaire de l'oeil du patient, comme une chirurgie réfractive, ou une correction d'aberration de l'oeil par une lentille de contact, un verre de lunette ou un implant oculaire.

Dans une variante du deuxième mode de réalisation du procédé selon l'invention, un utilisateur rentre dans des moyens de saisie la valeur de la correction qu'il souhaite simuler, correspondant par exemple à une valeur supposée de la myopie, de l'astigmatisme de l'oeil, ou une carte de phase comprenant aussi des ordres supérieurs d'aberrations de l'oeil tels que le coma, l'aberration sphérique, le trefoil par exemple. Les moyens de génération génèrent alors une consigne de modulation. L'utilisateur interagit avec le patient à l'aide de tests visuels, typiquement en demandant au patient s'il voit bien l'image test projetée sur la rétine de son oeil. Si le patient n'obtient pas de bons résultats aux tests visuels (c'est-à-dire s'il ne voit pas bien l'image test projetée sur la rétine de son oeil), l'utilisateur saisit une nouvelle valeur de la correction, jusqu'à ce que le patient obtienne des résultats satisfaisants aux tests. Si le patient obtient des résultats satisfaisants aux tests visuels (c'est-à-dire s'il voit bien l'image test projetée sur la rétine de son oeil), l'utilisateur peut en déduire que la valeur de la correction déterminée satisfait le besoin de correction des aberrations de l'oeil. Ainsi, le dispositif selon l'invention permet de simuler une correction, par exemple pour simuler une future opération d'ablation personnalisée de la cornée de l'oeil, ou pour réaliser un design de verres correcteurs adaptés à l'oeil.

Pour améliorer la qualité de la simulation de la correction, le dispositif selon l'invention comprend en outre des moyens pour mesurer des aberrations de l'oeil susceptibles de perturber le faisceau principal. Les moyens de mesure des aberrations comprennent les moyens de mesure MA, des moyens d'émission SRC d'un faisceau d'éclairage FE et un diaphragme d'éclairage APT. Lorsque les moyens d'émission SRC sont allumés, le faisceau d'éclairage est émis à travers le diaphragme APT, puis traverse une lentille L3, la lame séparatrice LM0, est réfléchi par la lame séparatrice LM1, puis est focalisé par la lentille L1 sur la rétine de l'oeil pour former un point source émettant un faisceau lumineux de diffusion FD. Le chemin optique du faisceau d'éclairage FE est illustré sur la figure 2 par deux lignes cadratin-point.

Le faisceau de diffusion FD émerge de l'oeil, puis parcours le chemin inverse du faisceau d'éclairage jusqu'à la lame séparatrice LM0. La lame séparatrice réfléchit le faisceau de diffusion, qui traverse alors le système optique S1 puis la lame séparatrice LM2. Le faisceau de diffusion parcourt ensuite un chemin optique allant de la lame LM2 jusqu'au moyens de mesure MA identique à celui décrit pour le faisceau secondaire FC. Le système optique S1 est agencé pour que le faisceau de diffusion FD soit un faisceau de lumière sensiblement collimatée lorsqu'il est intercepté par les moyens de modulation MD. Les moyens de modulation sont agencés pour moduler localement la phase du front d'onde du faisceau de diffusion FD selon la consigne de modulation. Les moyens de mesure MA permettent la mesure, dans le plan de mesure PA donné, de la phase du front de l'onde du faisceau de diffusion émis par le point source. Le chemin optique du faisceau de diffusion FD, qui va de la rétine de l'oeil jusqu'au moyens de mesure MA, est illustré sur la figure 2 par deux lignes pointillées. Pour que la mesure d'aberrations puisse être utilisée pour le faisceau principal FA, la longueur d'onde du faisceau de diffusion FD et celle du faisceau d'éclairage FE sont proches (à environ 100 nanomètres près) de celle du faisceau principal FA ou de préférence sensiblement égales à celle du faisceau principal FA, et sont donc de préférence situées dans le visible.

La lentille L1 est centrée sur un axe de mesure z correspondant à l'axe oculaire dans une position donnée de l'oeil, et est associée avec les lentilles L2 et L3 pour assurer la conjugaison optique entre la pupille de l'oeil, le plan du diaphragme d'éclairage APT et le plan de mesure PA des moyens de mesure MA.

Les moyens de traitement TRT, reliés aux moyens de mesure, établissent la cartographie en phase de l'onde FD émergente de l'oeil et calculent les aberrations de l'oeil. Une représentation de cette cartographie peut être affichée sur un écran SCR. Le calcul des aberrations de l'oeil peut être réalisé de manière classique, par exemple en ne modulant pas la phase du faisceau de diffusion par les moyens de modulation (consigne de modulation nulle), et en analysant la cartographie de phase du faisceau de diffusion FD. Les aberrations calculées sont mémorisées dans les moyens de traitement TRT.

De manière préférentielle, la mesure des aberrations de l'oeil (illustrée sur la figure 2 notamment par les faisceaux FE et FD) est réalisée en l'absence des faisceaux de mesure FC et principal FA. Lorsque l'on réalise le deuxième mode de réalisation de procédé selon l'invention (illustré sur la figure 1, notamment par les faisceaux FA et FC), il est possible mais pas préférable de continuer cette mesure d'aberrations, ceci afin de limiter le nombre de faisceaux lumineux interagissant avec l'oeil. De plus, il est préférable de ne pas utiliser en même temps les faisceaux de mesure FC et de diffusion FD car l'analyseur de phase MA ne peut de préférence analyser qu'un faisceau à la fois. Ainsi, si la source SI est allumée, la source SRC doit de préférence être éteinte et inversement. Une solution pour utiliser à la fois les faisceaux de mesure FC et de diffusion FD est de remplacer l'analyseur de phase MA par une lame séparatrice et par un premier et un deuxième analyseur de phase, la lame séparatrice dirigeant le faisceau secondaire FC vers le premier analyseur de phase et dirigeant le faisceau de diffusion FD vers le deuxième analyseur de phase, les premier et deuxième analyseurs de phase interagissant avec les moyens de traitement TRT de la même manière que l'analyseur MA. Cependant, cette solution n'est pas avantageuse en termes de coûts.

Ainsi, dans une autre variante du deuxième mode de réalisation de procédé selon l'invention, la consigne de modulation est calculée par les moyens de traitement en fonction des valeurs mesurées et/ou mémorisées des aberrations de l'oeil, de préférence pour compenser l'effet des aberrations sur le faisceau principal. Un utilisateur peut toujours ajuster la consigne de modulation via les moyens de saisie, par exemple pour affiner la simulation d'une correction statique solidaire de l'oeil du patient.

Cependant, la compensation des aberrations de l'oeil est limitée par les mouvements de l'oeil, mouvements latéraux (perpendiculaires à l'axe z) ou de rotation de l'axe oculaire, qui induisent des variations des aberrations de l'oeil à des fréquences d'évolution rapide (typiquement supérieure à 5 Hz). Le dispositif selon l'invention comprend donc de préférence des moyens pour mesurer les mouvements de l'oeil tels que décrits dans le document FR 2 866 551 et indépendants des moyens pour mesurer les aberrations de l'oeil. Les moyens de traitement TRT sont alors agencés pour calculer la consigne de modulation en fonction de valeurs mesurées des mouvements de l'oeil et de valeurs mesurées des aberrations de l'oeil.

Les moyens de mesure des mouvements de l'oeil comprennent des moyens d'éclairage de la pupille de l'oeil (typiquement deux diodes DEL non focalisées sur la pupille de l'oeil), un détecteur matriciel DET, et un objectif L4 formant l'image IMA de la pupille de l'oeil sur le détecteur. Avant d'être projetée sur le détecteur DET, l'image de la pupille de l'oeil traverse la lentille L1 et la lame séparatrice LM1, les lentilles L1 et L4 étant agencées pour conjuguer le plan de la pupille de l'oeil avec le plan du détecteur DET (comme illustré par le faisceau virtuel en ligne continue reliant l'oeil EYE et le détecteur DET sur les figures 1 et 2). Le détecteur est relié aux moyens de traitement TRT, qui, en suivant le mouvement de la pupille de l'oeil, mesurent le déplacement latéral de la pupille de l'oeil par rapport à une position prédéterminée.

Les moyens de mesure des mouvements de l'oeil peuvent comprendre en outre des moyens (non représentés) pour former au moins un faisceau focalisé dans le plan de la pupille de l'oeil et formant une tache lumineuse sur ladite pupille, la tache étant imagée sur le détecteur DET de la même manière que la pupille de l'oeil, les moyens de traitement étant agencés pour déterminer la rotation de l'oeil par rapport à une position prédéterminée, à partir des positions relatives des taches par rapport à l'image de la pupille sur détecteur.

Le simulateur comprend en outre un diaphragme de mesure DIA situé entre la lentille L2 et la lame séparatrice C1, et centré sur l'axe optique z des différents éléments optiques situés sur le chemin optique du faisceau d'éclairage FE, du faisceau de diffusion FD, et du faisceau secondaire FC. Les faisceaux de mesure FC et de diffusion FD sont sensiblement focalisés au centre du diaphragme de mesure DIA, et le diaphragme d'éclairage APT est décentré par rapport à l'axe optique z, de sorte que seul les faisceaux de mesure et de diffusion sont interceptés par les moyens de mesure MA, tous les autres flux lumineux parasites étant détourné des moyens de mesure MA. Le diaphragme de mesure permet notamment de filtrer tous les flux lumineux parasites réfléchis par la cornée de l'oeil et de tout autre dioptre situé entre la cornée et le diaphragme DIA.

Les moyens de modulation MD, les moyens de mesure MA, les moyens d'émission SI du faisceau lumineux de mesure FC et tous les moyens optiques situés sur le chemin du faisceau secondaire entre les moyens d'émission SI et les moyens de mesure MA, la source lumineuse VF et tous les moyens optiques situés sur le chemin du faisceau principal entre la source VF et la lame séparatrice LM1, les moyens d'émission SRC du faisceau d'éclairage FE et tous les moyens optiques situés sur le chemin du faisceau d'éclairage entre les moyens d'émission SRC et la lame séparatrice LM1, sont solidaires et montés sur une plate-forme PTF1 mobile le long de l'axe optique z de la lentille L1 dont le plan focal est situé sensiblement dans le plan de la pupille de l'oeil. La plate-forme PTF1 permet d'ajuster la focalisation de l'image de l'image test sur la rétine de l'oeil tout en conservant la conjugaison optique entre la pupille de l'oeil, le plan du diaphragme d'éclairage APT et le plan de mesure PA des moyens de mesure MA.

On remarque que le simulateur visuel selon l'invention peut fonctionner en envoyant dans l'oeil du patient uniquement le faisceau principal, tout en s'assurant grâce au faisceau secondaire que la modulation de phase effectuée sur le faisceau principal correspond à la consigne de modulation. Ainsi, le simulateur visuel selon l'invention permet d'assurer une bonne précision de simulation visuelle en limitant la gêne visuelle du patient. L'éclairage de l'oeil par les moyens d'émission SRC et/ou les diodes DEL pour la mesure des aberrations et/ou des mouvements de l'oeil permet de calculer la consigne en fonction des aberrations et/ou des mouvements de l'oeil, mais n'est qu'optionnel. En outre, compte tenu de la stabilité intrinsèque des aberrations de l'oeil dans le temps, une seule mesure des aberrations de l'oeil est nécessaire pour les connaître pendant la durée d'un examen ophtalmique utilisant le dispositif selon l'invention. Ainsi, les moyens d'émission SRC peuvent être éteints et la consigne de modulation peut être calculée en fonction de valeurs d'aberrations mémorisées.

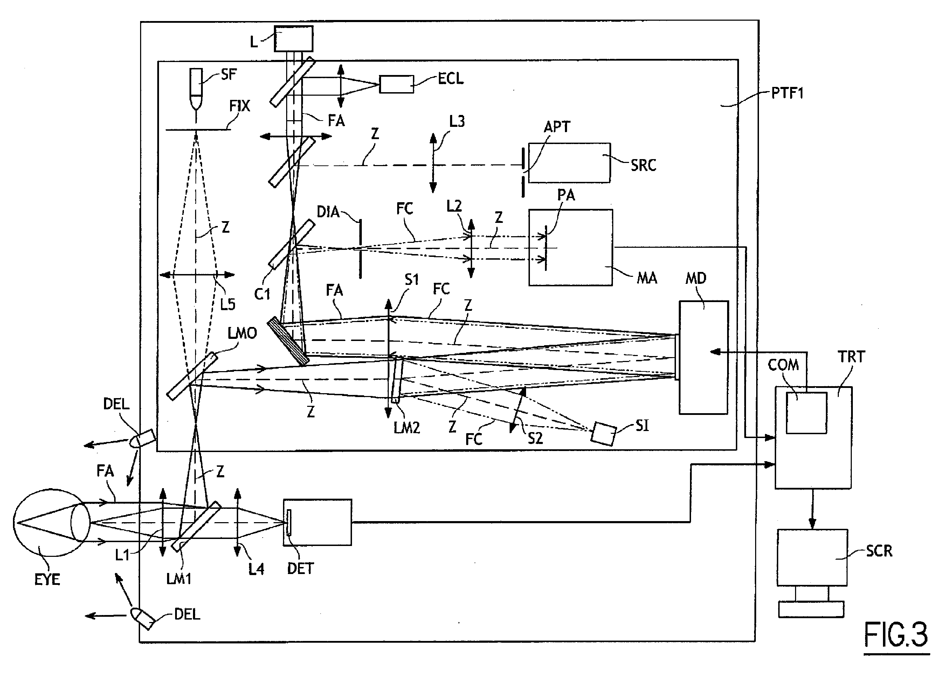

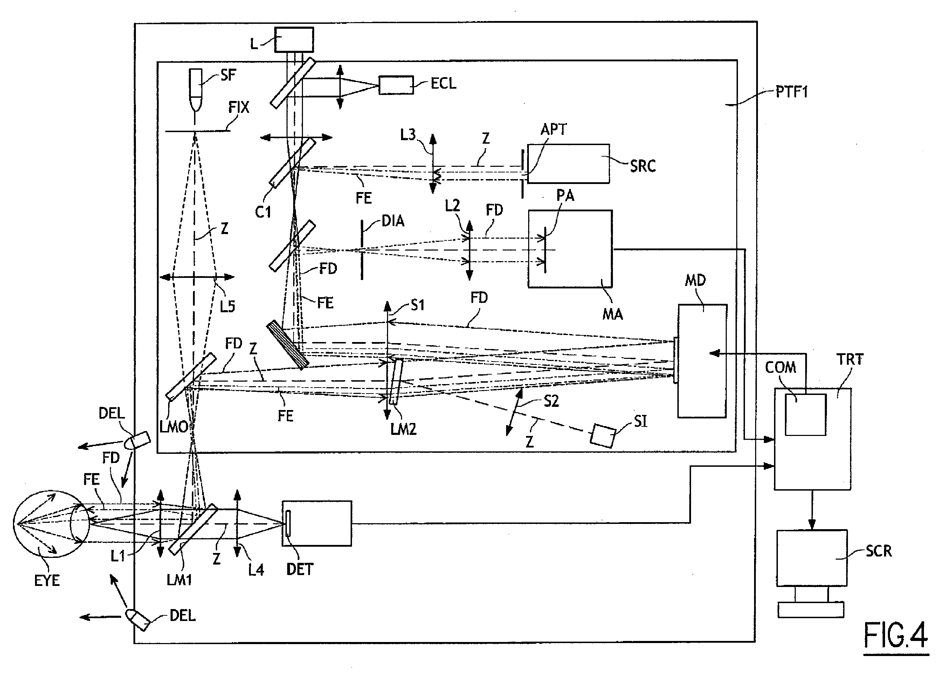

On va maintenant décrire, en référence aux figures 3 et 4, un dispositif de modulation de phase selon l'invention intégré dans un instrument d'imagerie rétinienne et mettant en oeuvre un procédé selon l'invention. On entend par système d'imagerie rétinienne tout type d'instrument permettant la visualisation de la rétine quel que soit son mode de fonctionnement. Cela peut être par exemple un système de caméra fond d'oeil, un système d'angiographie, un instrument de type Scanning Laser Ophtalmoscope (SLO), ou de type Optical Cohérence Tomography (OCT).

Cet instrument d'imagerie rétinienne selon l'invention sera décrit pour ses différences avec le simulateur visuel illustré sur les figures 1 et 2. En particulier, les agencements et les fonctions des moyens de mesure MA, du plan de mesure PA, des lentilles L1, et L2, de l'objectif L4, du diaphragme de mesure DIA, des lames séparatrices C1, LM0, LM1 et LM2, des systèmes optiques S1 et S2, des moyens de modulation de la phase MD, de l'unité de commande COM, des moyens de traitement TRT, de l'écran SCR, des moyens d'émission SI du faisceau secondaire FC, du détecteur DET, des diodes DEL et de l'oeil EYE sont les mêmes que dans le cas du simulateur de vision. Le chemin optique du faisceau secondaire FC (représenté sur la figure 3 par deux lignes cadratin-point-point) est donc le même que dans le cas du simulateur visuel.

Le faisceau principal ne consiste plus en une image projetée sur la rétine de l'oeil. En effet, la source lumineuse VF du simulateur est remplacée par un système d'éclairage ECL de la rétine de l'oeil EYE permettant d'éclairer la rétine sur une zone que l'on désire imager. Un détecteur L placé en sortie de l'instrument reçoit le faisceau principal FA, qui consiste en le faisceau rétro diffusé par la rétine et émergent de l'oeil. Une image de la rétine est formée sur le détecteur L, et peut être visualisée sur l'écran SCR. Le chemin optique du faisceau principal FA, allant de la rétine de l'oeil jusqu'au détecteur L est représenté sur la figure 3 par deux lignes en gras. La portion du chemin optique du faisceau principal, comprise entre l'oeil et la lame séparatrice C1, est commune avec le chemin optique du faisceau principal dans le cas du simulateur de vision illustré sur les figures 1 et 2. Les moyens de modulation MD sont donc agencés pour moduler la phase du faisceau principal.

Une voie de fixation, couplée au système par la lame séparatrice LM0, permet de fixer l'attention du patient dont l'oeil est analysé et de limiter les mouvements de l'oeil. Elle comprend une image éclairée FIX par une source lumineuse SF et des moyens d'imagerie L5 assurant la conjugaison optique de l'image avec la rétine, ou réglé de manière à ce que l'image soit vue avec une légère défocalisation myopique pour stimuler la désaccommodation.

L'instrument d'imagerie comprend également, comme dans le cas du simulateur de vision, les moyens de mesure des aberrations de l'oeil comprenant les moyens de mesure MA, les moyens d'émission SRC du faisceau d'éclairage FE, la lentille L3 et le diaphragme d'éclairage APT, disposés légèrement différemment mais avec les mêmes fonctions que dans le cas du simulateur de vision. En particulier, la conjugaison optique entre la pupille de l'oeil, le plan du diaphragme d'éclairage APT et le plan de mesure PA des moyens de mesure MA est assurée par l'association des lentilles L1, L2 et L3. Le faisceau d'éclairage est donc focalisé par la lentille L1 sur la rétine de l'oeil pour former un point source émettant un faisceau lumineux de diffusion FD. Le chemin optique du faisceau d'éclairage FE, allant des moyens d'émission SRC jusqu'à la rétine de l'oeil, est illustré sur la figure 4 par deux lignes cadratin-point. Typiquement, la zone de la rétine éclairée par.le système d'éclairage ECL est dix fois supérieure à la taille du point lumineux formé sur la rétine par le faisceau d'éclairage FE, car la mesure des aberrations nécessite une cartographie de la rétine de l'oeil mieux résolue que l'image de la rétine visualisée sur l'écran SCR.

Le chemin optique du faisceau de diffusion FD, qui va de la rétine de l'oeil jusqu'au moyens de mesure MA, est le même que dans le cas du simulateur visuel, et est illustré sur la figure 4 par deux lignes pointillées.

Les moyens de modulation MD, les moyens de mesure MA, les moyens d'émission SI du faisceau lumineux de mesure FC et tous les moyens optiques situés sur le chemin du faisceau secondaire entre les moyens d'émission SI et les moyens de mesure MA, la source lumineuse SF et tous les moyens optiques situés sur le chemin optique entre la source SF et la lame séparatrice LM1, les moyens d'émission SRC du faisceau d'éclairage FE et tous les moyens optiques situés sur le chemin du faisceau d'éclairage entre les moyens d'émission SRC et la lame séparatrice LM1, le système d'éclairage ECL et le détecteur L sont solidaires et montés sur une plate-forme PTF1 mobile le long de l'axe optique z de la lentille L1 dont le plan focal est situé sensiblement dans le plan de la pupille de l'oeil. La plate-forme PTF1 permet d'ajuster la focalisation du faisceau principal FA sur le détecteur L tout en conservant la conjugaison optique entre la pupille de l'oeil, le plan du diaphragme d'éclairage APT et le plan de mesure PA des moyens de mesure MA.

Le premier mode de réalisation de procédé selon l'invention peut être appliqué à l'instrument d'imagerie selon l'invention, et permet notamment de calibrer les moyens de détermination de la commande sans utiliser d'oeil artificiel, et en l'absence du faisceau principal FA.

De même, le deuxième mode de réalisation de procédé selon l'invention peut être appliqué à l'instrument d'imagerie selon l'invention, notamment pour s'assurer que la modulation de phase effectuée sur le faisceau principal correspond bien à la consigne de modulation. Comme pour le simulateur de vision selon l'invention, les moyens de traitement peuvent calibrer les moyens de détermination de la commande, et peuvent calculer la consigne de modulation en fonction de valeurs de correction saisies sur des moyens de saisie et/ou de valeurs mesurées ou mémorisées des aberrations de l'oeil et/ou de valeurs mesurées des mouvements de l'oeil, de préférence pour compenser sur le faisceau principal les aberrations et/ou les mouvements de l'oeil. L'instrument d'imagerie selon l'invention peut fonctionner en éclairant l'oeil du patient uniquement par les moyens d'éclairage ECL, tout en s'assurant grâce au faisceau secondaire que la modulation de phase effectuée sur le faisceau principal correspond à la consigne de modulation. L'éclairage de l'oeil par les moyens d'émission SRC pour la mesure d'aberrations de l'oeil, par les diodes DEL pour la mesure des mouvements de l'oeil, et par la source SF étant préférables mais optionnels. En outre, les moyens d'émission SRC peuvent être éteints et la consigne de modulation peut être calculée en fonction de valeurs d'aberrations mémorisées.

Contrairement au simulateur de vision, l'instrument d'imagerie décrit ci-dessus ne permet pas de simuler une correction de l'oeil, mais permet de s'assurer que la modulation du front d'onde du faisceau principal correspond bien à la consigne de modulation, ceci afin de donner à la phase du faisceau principal une forme permettant d'obtenir les performances optimales de l'instrument.

On va maintenir exposer une procédure pratique d'utilisation d'un instrument ophtalmique de simulation et/ou de correction selon l'invention :

   **Etape 1.** Une première étape concerne une mesure des aberrations avec la source d'éclairage de la rétine allumée, selon la procédure classique d'aberrométrie telle que divulguée dans le document FR 2 866 551 ;

**Etape 2.** Dans une seconde étape, on détermine la position de la pupille de l'oeil lors de la mesure des aberrations (étape 1) à l'aide de la caméra de poursuite de la pupille ;

**Etape 3.** Suit une étape d'extinction de la source d'éclairage de la rétine : le sujet voit la cible sans être gêné par une autre source ;

**Etape 4.** La source de faisceau lumineux secondaire est alors mis en route. Ce faisceau secondaire passe sur le miroir déformable avant d'être analysé par l'analyseur de front d'onde, et peut donc être utilisé dans la phase d'asservissement en boucle fermée du miroir déformable ;

**Etape 5.** Suit une application de la correction sur le miroir déformable en boucle fermée de la correction (ou de la carte de phase de simulation ou des deux). On utilise le faisceau lumineux secondaire pour cette étape, et ainsi aucune lumière n'est envoyée dans l'oeil du patient qui voit donc la cible sans être gêné. Pour être efficace, la correction (et/ou simulation) doit « suivre » le mouvement de la pupille de l'oeil du patient. La mesure du déplacement de la pupille est effectuée grâce au système de poursuite de pupille (« Pupil tracking »), et les informations de déplacement sont utilisées par l'algorithme de correction (et/ou simulation) pour générer sur le miroir déformable ladite correction (et/ou simulation) à la bonne position.

**Etape 6.** De façon concomitante à l'étape 5, le patient (et le praticien) peut mesurer subjectivement l'effet de la correction ou simulation sur la perception visuelle à l'aide de la cible de fixation, qui peut être un micro afficheur, - réalisé par exemple à partir d'une technologie OLED (Organic Light Emitting Diode : diode organique émettrice de lumière), LCD (Liquid Crystal Display : afficheur à cristaux liquides) ou MEMS (Micro Electro Mechanical Systems : microsystèmes électromécaniques) -, sur lequel peuvent être affichés différentes images ou tests visuels (tests d'acuité visuelle par exemple).

On peut éventuellement prévoir une étape **0** supplémentaire qui correspond à l'étape d'apprentissage et une étape **Obis** qui correspond à la maîtrise des aberrations du système optique de l'instrument ophtalmique, à l'aide du faisceau secondaire passant par le miroir déformable et analysé par l'analyseur. cette étape **Obis** de maîtrise peut consister en un asservissement du miroir déformable rapport à une consigne qui corrige l'ensemble des aberrations du système optique. Ainsi dans l'étape 1 précitée, l'analyseur mesure directement les aberrations oculaires.

Bien sûr, l'invention n'est pas limitée aux exemples qui viennent d'être décrits et de nombreux aménagements peuvent être apportés à ces exemples sans sortir du cadre de l'invention.

En particulier, on aurait pu aussi envisager le cas d'un instrument de traitement optique, identique à l'instrument d'imagerie illustré sur les figures 3 et 4, mais dans lequel le détecteur L est remplacé par une source laser. Dans ce cas, le faisceau principal consiste alors en le faisceau émis par la source laser et parcourt un chemin optique opposé à celui du faisceau principal de l'instrument d'imagerie selon l'invention.

De plus, les lentilles L1, L2, L3,... des instruments ophtalmiques décrits ci-dessus peuvent être remplacées par des moyens d'imagerie plus complexes, comprenant par exemple des combinaisons de lentilles.

**Revendications**

1. Dispositif de modulation de phase mis en oeuvre dans un instrument ophtalmique utilisant un faisceau lumineux principal (FA) interagissant avec un oeil (EYE), ce dispositif comprenant :

   - des moyens (MD) pour moduler la phase du front d'onde dudit faisceau lumineux principal (FA),
   - des moyens pour commander lesdits moyens de modulation de phase selon une consigne de modulation, et
   - des moyens pour analyser la modulation ainsi effectuée de la phase du front d'onde dudit faisceau lumineux principal (FA),

   **caractérisé en ce qu'**il comprend en outre des moyens pour émettre un faisceau lumineux secondaire (FC), des moyens pour diriger ledit faisceau lumineux secondaire (FC) le long d'un chemin optique vers lesdits moyens de modulation puis vers lesdits moyens d'analyse de front d'onde, ledit chemin optique ne passant par l'oeil,
   et **en ce que** les moyens de commande et les moyens d'analyse de front d'onde recevant ledit faisceau lumineux secondaire (FC) modulé coopèrent, au cours d'une phase dite d'apprentissage, pour fournir des données de réponse des moyens de modulation de phase à un ensemble de consignes de modulation prédéterminées.

2. Dispositif selon la revendication 1, dans lequel les moyens de modulation de phase comprennent un miroir déformable sous l'action d'une pluralité d'actionneurs, **caractérisé en ce que**, dans une phase d'apprentissage, les moyens de commande et les moyens d'analyse de front d'onde coopèrent pour mettre en mouvement successivement chaque actionneur et pour enregistrer, pour chaque actionneur, la réponse desdits moyens d'analyse de front d'onde.

3. Dispositif selon l'une des revendications 1 ou 2, **caractérisé en ce qu'**il comprend en outre des moyens (TRT) pour modifier la commande appliquée aux moyens de modulation (MD), de manière à réduire un écart entre la consigne de modulation et la modulation effectuée sur le faisceau lumineux secondaire (FC).

**4.** Dispositif selon l'une des revendications précédentes, **caractérisé en ce que** le chemin optique le long duquel le faisceau secondaire (FC) est guidé est interne à l'instrument ophtalmique.

**5.** Dispositif selon l'une des revendications précédentes, **caractérisé en ce qu'**une longueur d'onde du faisceau secondaire est sensiblement égale à une longueur d'onde du faisceau principal.

**6.** Dispositif selon l'une des revendications précédentes, **caractérisé en ce que** les moyens de modulation sont agencés pour moduler simultanément, selon une consigne de modulation, la phase du front d'onde du faisceau principal et la phase du front d'onde du faisceau lumineux secondaire (FC).

**7.** Dispositif selon l'une des revendications précédentes, **caractérisé en ce qu'**il comprend en outre des moyens (SF, FIX, VF) pour projeter sur la rétine de l'oeil une image d'une cible (VF, FIX).

**8.** Dispositif selon la revendication 7, **caractérisé en ce que** les rayons lumineux issus de la cible constituent le faisceau principal (FA).

**9.** Instrument ophtalmique, du type simulateur visuel, comprenant un dispositif de modulation de phase selon l'une revendications 7 ou 8, dans lequel les moyens de modulation soient commandés pour simuler sur le faisceau principal une correction statique solidaire de l'oeil, comme une chirurgie réfractive, ou une correction par une lentille de contact, un verre de lunette ou un implant oculaire.

**10.** Instrument d'imagerie rétinienne comprenant un dispositif de modulation de phase selon l'une des revendications 1 à 8, comprenant en outre des moyens pour éclairer la rétine de l'oeil (ECL), des moyens (L) pour détecter un faisceau lumineux émergent de l'oeil et pour former une image de la rétine, ledit faisceau émergent constituant le faisceau principal.

**11.** Instrument de traitement optique de la rétine de l'oeil, comprenant un dispositif de modulation de phase selon l'une quelconque des revendications 1 à 8, une source laser (L) pour l'émission du faisceau principal, et des moyens pour focaliser le faisceau principal sur la rétine de l'oeil.

**12.** Instrument d'aberrométrie de l'oeil, comprenant un dispositif de modulation de phase selon l'une quelconque des revendications 1 à 8, des moyens pour mesurer des aberrations de l'oeil susceptibles de perturber le faisceau principal, et des moyens pour calculer la consigne de modulation en fonction de valeurs mesurées des aberrations de l'oeil.

**13.** Instrument selon la revendication 12, **caractérisé en ce que** les moyens de mesure des aberrations de l'oeil comprennent des moyens d'émission (SRC) d'un faisceau d'éclairage (FE) pour former sur la rétine de l'oeil une source lumineuse secondaire, ladite source secondaire émettant un faisceau (FM) qui émerge de l'oeil, et des moyens pour guider ledit faisceau jusqu'à des moyens de mesure (MA) de la phase du front d'onde dudit faisceau.

**14.** Instrument ophtalmique comprenant un dispositif de modulation selon l'une des revendications 1 à 8, **caractérisé en ce qu'**il comprend en outre des moyens pour mesurer des mouvements de l'oeil, et des moyens pour calculer la consigne de modulation en fonction de valeurs mesurées des mouvements de l'oeil.

**15.** Instrument selon la revendication 14, **caractérisé en ce que** les moyens pour mesurer des mouvements de l'oeil comprennent des moyens pour mesurer un déplacement latéral de la pupille de l'oeil par rapport à une position prédéterminée.

**16.** Instrument selon l'une des revendications 14 ou 15, **caractérisé en ce que** les moyens pour mesurer les mouvements de la pupille de l'oeil comprennent des moyens d'éclairage (DEL) de la pupille de l'oeil, un détecteur matriciel (DET), et un objectif (L4) formant l'image de la pupille de l'oeil sur le détecteur.

**17.** Instrument selon l'une des revendications 14 à 16, **caractérisé en ce que** les moyens pour mesurer des mouvements de l'oeil comprennent des moyens pour mesurer une rotation de l'oeil par rapport à une position prédéterminée.

**18.** Instrument selon l'une des revendications 14 à 17, **caractérisé en ce que** les moyens pour mesurer les mouvements de l'oeil comprennent un détecteur matriciel (DET), un objectif (L4) formant l'image de la pupille de l'oeil sur le détecteur, des moyens pour former au moins un faisceau sensiblement focalisé dans le plan de la pupille de l'oeil et formant une tache lumineuse sur ladite pupille, et des moyens pour déterminer la rotation de l'oeil à partir des positions relatives de la tache par rapport à l'image de la pupille.

**19.** Procédé pour calibrer un appareil ophtalmique utilisant un faisceau lumineux principal (FA) interagissant avec un oeil (EYE) et dans lequel sont mises en oeuvre : (i) une modulation de la phase du front d'onde dudit faisceau lumineux principal (FA) par des moyens de modulation de phase et (ii) une analyse de ladite phase ainsi modulée du front d'onde (FA) par des moyens d'analyse de la phase, **caractérisé en ce qu'**il comprend, pendant une phase dite d'apprentissage :

    - une émission d'un faisceau lumineux secondaire (FC) qui est soumis à ladite modulation de phase puis à ladite analyse de phase, et

    - une séquence de commandes prédéterminées desdits moyens de modulation de phase, et d'enregistrements de données d'analyse de phase en réponse auxdites commandes de modulation,

et **en ce que** le faisceau lumineux secondaire (FC) est dirigé le long d'un chemin optique vers lesdits moyens de modulation et vers lesdits moyens d'analyse de la phase, ledit chemin optique ne passant pas par l'oeil.

20. Procédé selon la revendication 19, mis en oeuvre dans un appareil dans lequel les moyens de modulation de phase comprennent un miroir déformable sous l'action d'une pluralité d'actionneurs, **caractérisé en ce que** la séquence de commandes prédéterminées comprend des commandes pour mettre en mouvement successivement chaque actionneur et pour enregistrer, pour chaque actionneur, la réponse desdits moyens d'analyse de front d'onde.

21. Procédé selon l'une des revendications 19 ou 20, **caractérisé en ce qu'**il comprend en outre une modulation de la commande appliquée aux moyens de modulation (MD), de manière à réduire un écart entre la consigne de modulation et la modulation effectuée sur le faisceau secondaire (FC).

22. Procédé selon l'une des revendications 19 à 21, **caractérisé en ce qu'**il comprend en outre un guidage du faisceau (FC) le long d'un chemin optique de sorte que le faisceau secondaire n'interagisse pas avec l'oeil.

23. Procédé selon l'une des revendications 19 à 22, **caractérisé en ce qu'**il comprend en outre une modulation simultanée de la phase du front d'onde du faisceau principal et de la phase du front d'onde du faisceau secondaire.

24. Procédé selon l'une des revendications 19 à 23, mis en oeuvre dans un instrument ophtalmique comprenant une première source de génération d'un faisceau lumineux principal pour éclairer la rétine d'un oeil et une seconde source de génération d'un faisceau lumineux secondaire, **caractérisé en ce qu'**il comprend en outre :

    - une étape de mesure des aberrations du système optique dudit instrument ophtalmique, ladite première source d'éclairage de la rétine étant allumée,

    - une étape de détermination de la position de la pupille de l'oeil pendant ladite étape de mesure des aberrations,

    - une étape d'extinction de ladite première source d'éclairage de la rétine,

    - une étape de mise en route de ladite seconde source de génération du faisceau secondaire, ledit faisceau secondaire étant utilisé dans la phase d'apprentissage, et

    - une étape d'application de commandes de correction et/ou de simulation sur les moyens de modulation de phase.

25. Procédé selon la revendication 24, **caractérisé en ce qu'**il comprend en outre une mise en oeuvre d'une cible de fixation, telle qu'un micro-afficheur, sur laquelle des images ou tests visuels peuvent être affichés, ladite cible de fixation étant prévue pour procurer à un patient une mesure subjective de l'effet de la correction et/ou de la simulation.

26. Procédé selon l'une des revendications 24 ou 25, **caractérisé en ce qu'**il comprend en outre, préalablement à l'étape de mesure des aberrations, une étape initiale d'apprentissage et une étape de maîtrise des aberrations du système optique de l'instrument ophtalmique.

27. Procédé selon la revendication 26, **caractérisé en ce que** l'étape de maîtrise des aberrations du système optique comprend un asservissement des moyens de modulation de phase par rapport à une consigne déterminée de façon à corriger les aberrations dudit système optique.

**Patentansprüche**

1. Phasenmodulationsvorrichtung für ein ophthalmisches Instrument, bei dem ein Hauptlichtbündel (FA) verwendet wird, das mit einem Auge (EYE) interagiert, wobei diese Vorrichtung enthält:

    - eine Einrichtung (MD) zum Modulieren der Phase der Wellenfront des Hauptlichtbündels (FA),

    - eine Einrichtung zum Steuern der Phasenmodulationseinrichtung gemäß einem Modulationssollwert, und

    - eine Einrichtung zum Analysieren der so erfolgten Modulation der Phase der Wellenfront des Hauptlichtbündels (FA),

**dadurch gekennzeichnet, dass** sie ferner eine Einrichtung zum Emittieren eines Nebenlichtbündels (FC), eine Einrichtung zum Leiten des Nebenlichtbündels (FC) entlang eines Lichtwegs zu der Modulationseinrichtung und dann zu der Wellenfrontanalyseeinrichtung, wobei der Lichtweg nicht durch das Auge tritt, aufweist,

und dass die Steuereinrichtung und die Wellenfrontanalyseeinrichtung, die das modulierte Nebenlichtbündel (FC) aufnimmt, während einer sogenannten Lernphase zusammenwirken, um Daten über die Reaktion der Phasenmodulationseinrichtung auf eine Menge von vorbestimmten Modulationssollwerten bereitzustellen.

2. Vorrichtung nach Anspruch 1, wobei die Phasenmodulationseinrichtung einen unter der Wirkung einer Mehrzahl von Betätigungsgliedern verformbaren Spiegel aufweist, **dadurch gekennzeichnet, dass** in einer Lernphase die Steuereinrichtung und die Wellenfrontanalyseeinrichtung zusammenwirken, um nacheinander jedes Betätigungsglied in Bewegung zu versetzen und um für jedes Betätigungsglied die Reaktion der Wellenfrontanalyseeinrichtung aufzuzeichnen.

3. Vorrichtung nach einem der Ansprüche 1 oder 2, **dadurch gekennzeichnet, dass** sie ferner eine Einrichtung (TRT) zum Ändern des der Modulationseinrichtung (MD) erteilten Befehls aufweist, so dass eine Abweichung zwischen dem Modulationssollwert und der am Nebenlichtbündel (FC) erfolgten Modulation vermindert wird.

4. Vorrichtung nach einem der vorangehenden Ansprüche, **dadurch gekennzeichnet, dass** der Lichtweg, entlang welchem das Nebenbündel (FC) geführt wird, innerhalb des ophthalmischen Instruments liegt.

5. Vorrichtung nach einem der vorangehenden Ansprüche, **dadurch gekennzeichnet, dass** eine Wellenlänge des Nebenbündels im Wesentlichen gleich einer Wellenlänge des Hauptbündels ist.

6. Vorrichtung nach einem der vorangehenden Ansprüche, **dadurch gekennzeichnet, dass** die Modulationseinrichtung dazu vorgesehen ist, zugleich die Phase der Wellenfont des Hauptbündels und die Phase der Wellenfont des Nebenlichtbündels (FC) gemäß einem Modulationssollwert zu modulieren.

7. Vorrichtung nach einem der vorangehenden Ansprüche, **dadurch gekennzeichnet, dass** sie ferner eine Einrichtung (SF, FIX, VF) aufweist, um ein Bild eines Ziels (VF, FIX) auf die Netzhaut des Auges zu projizieren.

8. Vorrichtung nach Anspruch 7, **dadurch gekennzeichnet, dass** die von dem Ziel stammenden Lichtstrahlen das Hauptbündel (FA) bilden.

9. Ophthalmisches Instrument vom Typ visueller Simulator, enthaltend eine Phasenmodulationsvorrichtung nach einem der Ansprüche 7 oder 8, wobei die Modulationseinrichtung so gesteuert wird, dass über das Hauptbündel eine fest mit dem Auge verbundene statische Korrektur, wie etwa eine refraktive Chirurgie, oder eine Korrektur mittels einer Kontaktlinse, einem Brillenglas oder einem Augenimplantat simuliert wird.

10. Instrument zur Netzhautabbildung, enthaltend eine Phasenmodulationsvorrichtung nach einem der Ansprüche 1 bis 8, ferner enthaltend eine Einrichtung zum Ausleuchten der Netzhaut des Auges (ECL), eine Einrichtung (L) zum Detektieren eines Lichtbündels, das aus dem Auge austritt, und zum Bilden eines Bildes der Netzhaut, wobei das austretende Lichtbündel das Hauptbündel darstellt.

11. Instrument zur optischen Behandlung der Netzhaut des Auges, enthaltend eine Phasenmodulationsvorrichtung nach einem der Ansprüche 1 bis 8, eine Laserquelle (L) zum Emittieren des Hauptbündels und eine Einrichtung zum Fokussieren des Hauptbündels auf der Netzhaut des Auges.

12. Instrument zur Aberrometrie des Auges, enthaltend eine Phasenmodulationsvorrichtung nach einem der Ansprüche 1 bis 8, eine Einrichtung zum Messen der Aberrationen des Auges, die dazu geeignet ist, das Hauptbündel zu stören, und eine Einrichtung zum Berechnen des Modulationssollwerts in Abhängigkeit von Messwerten der Aberrationen des Auges.

13. Instrument nach Anspruch 12, **dadurch gekennzeichnet, dass** die Einrichtung zum Messen der Aberrationen des Auges eine Emissionseinrichtung (SRC) zum Emittieren eines Ausleuchtbündels (FE) aufweist, um auf der Netzhaut des Auges eine Nebenlichtquelle zu bilden, wobei die Nebenquelle ein Bündel (FM) emittiert, das aus dem Auge austritt, sowie eine Einrichtung zum Führen des Lichtbündels bis zur Messeinrichtung (MA) zum Messen der Phase der Wellenfront des Lichtbündels.

14. Ophthalmisches Instrument, enthaltend eine Modulationsvorrichtung nach einem der Ansprüche 1 bis 8, **dadurch gekennzeichnet, dass** es ferner eine Einrichtung zum Messen der Bewegungen des Auges und eine Einrichtung zum Berechnen des Modulationssollwertes in Abhängigkeit von den Messwerten der Bewegungen des Auges aufweist.

15. Instrument nach Anspruch 14, **dadurch gekenn-**

**zeichnet, dass** die Messeinrichtung zum Messen der Bewegungen des Auges eine Messeinrichtung zum Messen einer seitlichen Verlagerung der Pupille des Auges bezüglich einer vorbestimmten Position aufweist.

16. Instrument nach einem der Ansprüche 14 oder 15, **dadurch gekennzeichnet, dass** die Messeinrichtung zum Messen der Bewegungen der Pupille des Auges eine Ausleuchteinrichtung (DEL) zum Ausleuchten der Pupille des Auges, einen Matrixdetektor (DET) und ein Objektiv (L4) aufweist, das das Bild der Augenpupille auf dem Detektor bildet.

17. Instrument nach einem der Ansprüche 14 bis 16, **dadurch gekennzeichnet, dass** die Messeinrichtung zum Messen der Bewegungen des Auges eine Messeinrichtung zum Messen einer Drehung des Auges bezüglich einer vorbestimmten Position aufweist.

18. Instrument nach einem der Ansprüche 14 bis 17, **dadurch gekennzeichnet, dass** die Messeinrichtung zum Messen der Bewegungen des Auges einen Matrixdetektor (DET), ein Objektiv (L4), das das Bild der Augenpupille auf dem Detektor bildet, eine Einrichtung zum Bilden zumindest eines Lichtbündels, das im Wesentlichen in die Ebene der Augenpupille fokussiert wird und einen Lichtfleck auf der Pupille bildet, und eine Einrichtung zum Ermitteln der Drehung des Auges ausgehend von relativen Positionen des Lichtflecks bezüglich des Bildes der Pupille aufweist.

19. Verfahren zum Kalibrieren eines ophthalmischen Geräts, bei dem ein Hauptlichtbündel (FA) verwendet wird, das mit einem Auge (EYE) interagiert, und bei dem: (i) eine Modulation der Phase der Wellenfront des Hauptlichtbündels (FA) durch die Phasenmodulationseinrichtung und (ii) eine Analyse der so modulierten Phase der Wellenfront (FA) durch die Phasenanalyseeinrichtung erfolgen, **dadurch gekennzeichnet, dass** es während der sogenannten Lernphase umfasst:

- eine Emission eines Nebenlichtbündels (FC), welches der Phasenmodulation und dann der Phasenanalyse unterzogen wird, und
- eine Abfolge von vorbestimmten Befehlen an die Phasenmodulationseinrichtung und von Aufzeichnungen von Phasenanalysedaten in Reaktion auf die Modulationsbefehle,

und dass das Nebenlichtbündel (FC) entlang eines Lichtwegs zu der Modulationseinrichtung und zu der Phasenanalyseeinrichtung geleitet wird, wobei der Lichtweg nicht durch das Auge verläuft.

20. Verfahren nach Anspruch 19, das mit einem Gerät durchgeführt wird, in welchem die Phasenmodulationseinrichtung einen unter der Wirkung einer Mehrzahl von Betätigungsgliedern verformbaren Spiegel aufweist, **dadurch gekennzeichnet, dass** die Abfolge von vorbestimmten Befehlen Befehle umfasst, um nacheinander jedes Betätigungsglied in Bewegung zu versetzen und um für jedes Betätigungsglied die Reaktion der Wellenfrontanalyseeinrichtung aufzuzeichnen.

21. Verfahren nach einem der Ansprüche 19 oder 20, **dadurch gekennzeichnet, dass** es ferner eine Modulation des der die Modulationseinrichtung (MD) erteilten Befehls umfasst, so dass eine Abweichung zwischen dem Modulationssollwert und der am Nebenlichtbündel (FC) erfolgten Modulation vermindert wird.

22. Verfahren nach einem der Ansprüche 19 bis 21, **dadurch gekennzeichnet, dass** es ferner eine Führung des Lichtbündels (FC) entlang eines Lichtwegs umfasst, so dass das Nebenlichtbündel nicht mit dem Auge interagiert.

23. Verfahren nach einem der Ansprüche 19 bis 22, **dadurch gekennzeichnet, dass** es ferner eine gleichzeitige Modulation der Phase der Wellenfront des Hauptlichtbündels und der Phase der Wellenfront des Nebenlichtbündels umfasst.

24. Verfahren nach einem der Ansprüche 19 bis 23, das mit einem ophthalmischen Instrument durchgeführt wird, das eine erste Quelle zum Erzeugen eines Hauptlichtbündels enthält, um die Netzhaut eines Auges auszuleuchten, sowie eine zweite Quelle zum Erzeugen eines Nebenlichtbündels, **dadurch gekennzeichnet, dass** es umfasst:

- einen Schritt des Messens der Aberrationen des optischen Systems des ophthalmischen Instruments, wobei die erste Quelle zum Ausleuchten der Netzhaut eingeschaltet ist,
- einen Schritt des Bestimmens der Position der Pupille des Auges während des Schrittes des Messens von Aberrationen,
- einen Schritt des Ausschaltens der ersten Quelle zum Ausleuchten der Netzhaut,
- einen Schritt des Ingangsetzens der zweiten Quelle zum Erzeugen des Nebenlichtbündels, wobei das Nebenlichtbündel bei der Lernphase verwendet wird, und
- einen Schritt des Erteilens von Korrektur- und/oder Simulationsbefehlen an die Phasenmodulationseinrichtung.

25. Verfahren nach Anspruch 24, **dadurch gekennzeichnet, dass** es ferner ein Einsetzen eines Fixierungsziels umfasst, wie etwa einer Mikroanzeige, worauf Bilder bzw. visuelle Tests angezeigt werden können, wobei das Fixierungsziel dazu vorgesehen ist, einem Patienten eine subjektive Messung der Wirkung der Korrektur und/oder der Simulation bereitzustellen.

26. Verfahren nach einem der Ansprüche 24 oder 25, **dadurch gekennzeichnet, dass** es ferner vor dem Schritt des Messens von Aberrationen einen anfänglichen Lernschritt und einen Schritt des Beherrschens von Aberrationen des optischen Systems des ophthalmischen Instruments umfasst.

27. Verfahren nach Anspruch 26, **dadurch gekennzeichnet, dass** der Schritt des Beherrschens von Aberrationen des optischen Systems eine Regelung der Phasenmodulationseinrichtung bezüglich eines vorbestimmten Sollwerts umfasst, so dass die Aberrationen des optischen Systems korrigiert werden.

**Claims**

1. Phase modulation device implemented in an ophthalmic instrument using a main light beam (FA) interacting with an eye (EYE), this device comprising:

   - means (MD) for modulating the phase of the wave front of said main light beam (FA),
   - means for controlling said phase modulation means following a modulation instruction, and
   - means for analysing the thus-performed modulation of the phase of the wave front of said main light beam (FA),

   **characterized in that** it comprises moreover means for emitting a secondary light beam (FC), means for directing said secondary light beam (FC) along an optical path to said modulation means then to said wave front analysis means, and **in that** said optical path does not pass through the eye, and **in that** the control means and wave front analysis means receiving said modulated secondary light beam (FC) cooperate, during a stage called a learning stage, to supply data regarding the response of the phase modulation means to a set of predetermined modulation instructions.

2. Device according to claim 1, in which the phase modulation means comprise a deformable mirror actuated by a plurality of actuators, **characterized in that**, in a learning stage, the wave front analysis means and control means cooperate in order to set each actuator successively in motion and to store, for each actuator, the response of said wave front analysis means.

3. Device according to one of claims 1 or 2, **character-**

**ized in that** it comprises moreover means (TRT) for modifying the control applied to the modulation means (MD), in order to reduce a discrepancy between the modulation instruction and the modulation carried out on the secondary light beam (FC).

4. Device according to one of the previous claims, **characterized in that** the optical path along which the secondary beam (FC) is guided is internal to the ophthalmic instrument.

5. Device according to one of the previous claims, **characterized in that** a wavelength of the secondary beam is approximately equal to a wavelength of the main beam.

6. Device according to one of the previous claims, **characterized in that** the modulation means are arranged in order to modulate, according to a modulation instruction, the phase of the wave front of the main beam and the phase of the wave front of the secondary light beam (FC) simultaneously.

7. Device according to one of the previous claims, **characterized in that** it comprises moreover means (SF, FIX, VF) for projecting an image of a target (VF, FIX) onto the retina of the eye.

8. Device according to claim 7, **characterized in that** the light rays originating from the target constitute the main beam (FA).

9. Ophthalmic instrument, of the visual simulator type, comprising a phase modulation device according to one of claims 7 or 8, in which the modulation means are controlled in order to simulate on the main beam a static correction integral with the eye, such as refractive surgery, or a correction by a contact lens, a spectacles lens or an ocular implant.

10. Retinal imaging instrument comprising a phase modulation device according to one of claims 1 to 8, comprising moreover means for illuminating the retina of the eye (ECL), means (L) for detecting a light beam emerging from the eye and for forming an image of the retina, said emergent beam constituting the main beam.

11. Instrument for optical treatment of the retina of the eye, comprising a phase modulation device according to any one of claims 1 to 8, a laser source (L) for emitting the main beam, and means for focussing the main beam onto the retina of the eye.

12. Instrument for aberrometry of the eye, comprising a phase modulation device according to any one of claims 1 to 8, means for measuring aberrations of the eye liable to interfere with the main beam, and means for calculating the modulation instruction as a function of measured values of the aberrations of the eye.

13. Instrument according to claim 12, **characterized in that** the means for measuring aberrations of the eye comprise means of emission (SRC) of an illumination beam (FE) in order to form a secondary light source on the retina of the eye, said secondary source emitting a beam (FM) which emerges from the eye, and means for guiding said beam to means of measurement (MA) of the phase of the wave front of said beam.

14. Ophthalmic instrument comprising a modulation device according to one of claims 1 to 8, **characterized in that** it comprises moreover means for measuring movements of the eye, and means for calculating the modulation instruction as a function of measured values of the movements of the eye.

15. Instrument according to claim 14, **characterized in that** the means for measuring movements of the eye include means for measuring a lateral displacement of the pupil of the eye with respect to a predetermined position.

16. Instrument according to one of claims 14 or 15, **characterized in that** the means for measuring the movements of the pupil of the eye comprise means of illuminating (DEL) the pupil of the eye, an array detector (DET), and an objective (L4) forming the image of the pupil of the eye on the detector.

17. Instrument according to one of claims 14 to 16, **characterized in that** the means for measuring movements of the eye comprise means for measuring a rotation of the eye with respect to a predetermined position.

18. Instrument according to one of claims 14 to 17, **characterized in that** the means for measuring the movements of the eye comprise an array detector (DET), an objective (L4) forming the image of the pupil of the eye on the detector, means for forming at least one beam substantially focussed in the plane of the pupil of the eye and forming a light spot on said pupil, and means for determining the rotation of the eye from the relative positions of the spot with respect to the image of the pupil.

19. Process for calibrating an ophthalmic device using a main light beam (FA) interacting with an eye (EYE) in which are implemented: (i) a phase modulation of the wave front of said main light beam (FA) by phase modulation means and (ii) an analysis of said wave front phase (FA) modulated in this way by phase analysis means, **characterized in that** it comprises, during a stage called a learning stage:

- emitting a secondary light beam (FC) which is subjected to said phase modulation then to said phase analysis, and
- a sequence of predetermined controls of said phase modulation means, and storage of phase analysis data in response to said modulation controls,

and **in that** the secondary light beam (FC) is directed along an optical path to said modulation means and to said phase analysis means, and **in that** said optical path does not pass through the eye.

20. Process according to claim 19, implemented in a device in which the phase modulation means comprise a mirror which can be deformed by the action of a plurality of actuators, **characterized in that** the sequence of predetermined controls comprises controls for successively starting each actuator to move and for storing, for each actuator, the response of said wave front analysis means.

21. Process according to one of claims 19 or 20, **characterized in that** it comprises moreover a modulation of the control applied to the modulation means (MD), so as to reduce a discrepancy between the modulation instruction and the modulation carried out on the secondary beam (FC).

22. Process according to one of claims 19 to 21, **characterized in that** it comprises moreover guiding the beam (FC) along an optical path so that the secondary beam does not interact with the eye.

23. Process according to one of claims 19 to 22, **characterized in that** it comprises moreover a simultaneous modulation of the phase of the wave front of the main beam and the phase of the wave front of the secondary beam.

24. Process according to one of claims 19 to 23, implemented in an ophthalmic instrument comprising a primary source for generating a main light beam for illuminating the retina of an eye and a second source for generating a secondary light beam, **characterized in that** it comprises moreover:

- a step of measuring the aberrations of the optical system of said ophthalmic instrument, said primary source of illumination of the retina being switched on,
- a step of determining the position of the pupil of the eye during said step of measurement of the aberrations,
- a step of switching off said primary source of illumination of the retina,

- a step of starting said second source for generating the secondary beam, said secondary beam being used in the learning stage, and
- a step of applying correction and/or simulation controls to the phase modulation means.

**25.** Process according to claim 24, **characterized in that** it comprises moreover implementing a fixation target, such as a micro-display, on which visual tests or images can be displayed, said fixation target being provided to give a patient a subjective measure of the effect of the correction and/or the simulation.

**26.** Process according to one of claims 24 or 25, **characterized in that** it comprises moreover, prior to the step of measuring the aberrations, an initial learning step and a step of overcoming the aberrations of the optical system of the ophthalmic instrument.

**27.** Process according to claim 26, **characterized in that** the step of overcoming the aberrations of the optical system comprises an enslavement of the phase modulation means with respect to a determined instruction, in order to correct the aberrations of said optical system.

FIG.1

EP 2 134 248 B1

FIG.2

FIG.3

EP 2 134 248 B1

21

FIG.4

FIG.5

EP 2 134 248 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2866551

- WO 2005089635 A